# EUROPÄISCHE PATENTSCHRIFT

(11) **EP 3 211 920 B1**
(45) Veröffentlichungstag und Bekanntmachung des Hinweises auf die Patenterteilung: **03.06.2020**
(21) Anmeldenummer: 17157227.4
(22) Anmeldetag: 21.02.2017
(51) Int. Cl.: H04R 25/00

(54) **VERFAHREN UND VORRICHTUNG ZUR KONFIGURATION EINES NUTZERSPEZIFISCHEN HÖRSYSTEMS**
METHOD AND DEVICE FOR CONFIGURING A USER-SPECIFIC HEARING SYSTEM
PROCÉDÉ ET DISPOSITIF DESTINÉS À LA CONFIGURATION D'UN APPAREIL AUDITIF SPÉCIFIQUE À L'UTILISATEUR

(30) Priorität: 25.02.2016 DE 102016103297
(43) Veröffentlichungstag der Anmeldung: 30.08.2017
(73) Patentinhaber: audiosus GmbH, 91484 Sugenheim (DE)
(72) Erfinder: Stinn, Christoph, 91484 Sugenheim (DE); Möckel, Peter, 98617 Sülzfeld (DE)
(74) Vertreter: Engel, Christoph Klaus

(56) Entgegenhaltungen:
- EP-A2- 1 538 868
- WO-A1-2004/054318
- WO-A1-2011/014906
- US-A1- 2002 040 254
- US-A1- 2006 140 418
- US-A1- 2010 310 101

## Beschreibung

Die vorliegende Erfindung bezieht sich auf ein Verfahren zur Konfiguration eines nutzerspezifischen Hörsystems. Ein solches Hörsystem umfasst herkömmlich eine Empfangseinheit zum Empfang von Schallereignissen, eine Verstärkungseinheit zur frequenzabhängig variablen Verstärkung der empfangenen Schallereignisse und eine Wiedergabeeinheit zur Wiedergabe der verstärkten Schallereignisse an das Hörorgan eines Nutzers.

Um Nutzern mit verminderter Hörfähigkeit ein besseres Hören zu ermöglichen, werden heutzutage komplexe Hörsysteme angeboten, die eine Verstärkung von Schallereignissen vornehmen und die verstärkten Schallsignale dem Nutzer einseitig oder beidseitig am Ohr zur Verfügung stellen. Zur Optimierung des unterstützten Hörens ist es dabei erforderlich, dass die Verstärkungscharakteristik des Hörsystems auf den nutzerspezifischen Hörschaden angepasst wird, da üblicherweise eine frequenzabhängige Verschlechterung des natürlichen Hörens gegeben ist, sodass eine lineare Verstärkung sämtlicher Schallereignisse nicht zu dem gewünschten Ergebnis führen würde. Moderne Hörsysteme gestatten eine frequenzabhängige Verstärkung durch eine Parametereinstellung, die während einer initialen Konfiguration des Hörsystems vorzugsweise von einem Hörgeräteakustiker vorgenommen wird.

In der EP 1 746 859 B1 ist ein Verfahren zum Konfigurieren eines Hörgerätes für eine Person beschrieben. Dazu werden ein Testtöne erzeugendes Audiometer und ein an das Audiometer angeschlossener, vom Patienten getragener offener Kopfhörer zum Übertragen der Testtöne verwendet. Während der Konfiguration trägt der Nutzer sein Hörgerät und die offenen Kopfhörer. Das Hörgerät wird konfiguriert, um das vom Audiometer über die Kopfhörer abgegebene Signal bis zum Erreichen der Hörschwelle des Patienten frequenzspezifisch zu verstärken. In der Praxis hat sich allerdings gezeigt, dass die Konfiguration eines Hörsystems durch eine frequenzspezifische Verstärkung jeweils bis zum Erreichen der Hörschwelle des Nutzers nicht zu befriedigenden Ergebnissen führt. Während der Konfiguration des Hörsystems ist es für den Nutzer schwierig, das Auftreten eines verstärkten Signals an der Hörschwelle präzise zu identifizieren.

Aus der DE 10 2007 054 152 A1 ist ein Verfahren zur Beurteilung des Hörvermögens von Menschen und zur Programmierung von Hörhilfen bekannt. Eine Testperson wird dabei in einem Testraum beabstandet von einem Lautsprecherarray positioniert, woraufhin von dem Lautsprecherarray Testtöne wiedergegeben werden, die mittels Wellenfeldsynthese virtuelle Tonquellen räumlich darstellen. Die Testperson soll sich nachfolgend über die empfundene Lautstärke der gehörten Testtöne äußern, um so ein Hörbild der Testperson zu bestimmen. Das subjektive Empfinden der Testpersonen bezüglich der Testtöne wird mit dem Hörempfinden eines Normalhörenden verglichen und ein Hörbild des Testpatienten bestimmt.

Die DE 10 2011 104 536 A1 beschreibt ein Verfahren und eine Vorrichtung zum Auswählen und Konfigurieren eines Hörgerätes für einen Patienten. Der Patient wird zunächst einem allgemeinen Screening zum Feststellen seiner Hörschwelle unterworfen. Nachfolgend wir der Patient mit einem Hörgerät ausgerüstet und in einem freien Schallfeld mit festen Signalen ansteigender Lautstärke beschallt, bis er zu erkennen gibt, Signale wahrgenommen zu haben. Das Hörgerät wird anhand dieser Erkenntnisse voreingestellt. Anschließend werden am Hörgerät Schwellenwerte für die Hörschwelle, den angenehmen Hörbereich und dem Unbehaglichkeitsbereich eingestellt.

In der US 6,201,875 B1 ist ein Verfahren zum Anpassen eines Hörgerätes beschrieben. Hierbei wird unter anderem ein Stimulisatz von Impulsen bereitgestellt, der eine Vielzahl Lautstärkelevel für eine Vielzahl der Frequenzen umfasst. In einem Verfahrensschritt wird jedes Lautstärkelevel für jede Frequenz bzgl. der wahrgenommenen Gleichheit verglichen. Die zu vergleichenden Töne werden durch das Hörgerät und nicht durch eine Umgebungsbedingungen simulierende Lautsprecheranordnung bereitgestellt.

Die WO 2004/054318 A1 zeigt ein Verfahren zur Anpassung eines transportierbaren Kommunikationsgeräts für hörbehinderte Nutzer. Dem Nutzer werden zwei Stimulilevel unterschiedlicher Frequenzbänder eines Wahrnehmungsfrequenzbandes präsentiert, deren Lautheit er beurteilen und vergleichen soll.

In der EP 1 538 868 A2 ist ein Audio-Verstärkungsgerät sowie ein Verfahren zum Einstellen diese Gerätes mittels Anpassung einer frequenzabhängigen Verstärkung gezeigt. Ein Verfahrensschritt sieht vor, dass mehrere Frequenzen einem Ausgangswandler bereitgestellt werden. In einem weiteren Verfahrensschritt werden die Stimulilevel bzw. -pegel jeder Frequenz derart angepasst, dass sie einem vordefinierten Lautheitslevel oder einer Detektionsschwelle eines Nutzers entsprechen. Dem Nutzer wird ein Stimuliset mit schmaler Bandbreite vorgestellt. Der Nutzer beurteilt die Lautheit der Stimuli. Die Lautheit jedes Stimulus wird mit der Lautheit eines Referenzstimulus verglichen, wobei der Referenzstimulus eine gewählte bestimmte Frequenz aufweist. Dem Nutzer werden verschiedene Stimuli vorgespielt, welche mit dem konstant bleibenden Referenzstimulus verglichen werden und gemäß den Einschätzungen des Nutzers in ihrem Pegel angeglichen werden, bis die Lautheit gleich der Lautheit des Referenzstimulus ist.

Die US 2006/0140418 A1 beschreibt eine Methode zur Kompensation von Audiofrequenzgangeigenschaften eines transportierbaren Soundsystems. Es werden ein Referenzton (1000 Hz) und ein dazugehöriger Schalldruckpegel (0 bis 120 dB) festgelegt. Der Referenzton und ein reiner Ton einer anderen Frequenz werden abwechselnd von beiden Hörgeräten wiedergegeben, wobei man einen Schalldruckpegel gleicher Lautheit für den Referenzton und den reinen Ton erhält.

Die US 2002/0040254 A1 zeigt ein Gerät sowie eine Methode, welche einen interaktiven Prozess zum Verbessern des Hörerlebnisses nutzen. Der Klang des linken und des rechten Kopfhörers werden durch den Nutzer angeglichen, wobei eine Frequenz erst von einem und dann von dem anderen Kopfhörer wiedergegeben werden und diese anschließend zentriert werden.

In der US 2010/0310101 A1 werden ein Verfahren sowie ein Gerät zum akustischen Anpassen einer Hörhilfe beschrieben. Dem Nutzer wird eine Vielzahl hörbarer Töne mit jeweils einer vorbestimmten Frequenz über Kopfhörer wiedergegeben. Die Töne weisen spezifische Schalldrücke je Ohr auf. Der Nutzer verändert den Schalldruck dermaßen, dass die wahrgenommene Richtung der Töne vor dem Nutzer liegt.

Die WO 2011/014906 A1 betrifft ein Verfahren zur Anpassung eines Tonverarbeitungsgerätes bzw. Hörsystems für einen Nutzer unter Verwendung von verbesserten Klängen, wobei dem Nutzer ein oder mehrere Geräusche bzw. Noten von dem Tonverarbeitungsgerät präsentiert werden. Jede Note umfasst eine Sammlung von zwei oder mehr harmonisch verwandten Tönen, welche spektral um die interessierende Frequenz positioniert sind. Weiterhin wird beschrieben, dass der Nutzer eine Sequenz von musikalischen Tönen verschiedener Frequenzen hört und instruiert ist, die Lautheit der einzelnen Noten anzupassen, bis diese gleich laut und angenehm sind.

Die Aufgabe der vorliegenden Erfindung besteht ausgehend vom Stand der Technik darin, ein verbessertes Verfahren bereitzustellen, das eine Konfiguration eines nutzerspezifischen Hörsystems gestattet, sodass das Hörerlebnis des Nutzers nach der Konfiguration optimiert ist. Das Verfahren soll möglichst einfach gestaltet sein, um eine Anwendung in üblichen Betrieben von Hörgeräteakustikern zu ermöglichen und um den Nutzer während der Konfiguration nicht zu überfordern.

Diese Aufgabe wird durch ein Verfahren gemäß dem beigefügten Anspruch 1 gelöst.

Die Erfindung basiert zunächst auf dem Ansatz, dass zwei benachbarte Schallereignisse verglichen werden, die dem Nutzer nacheinander wiedergegeben werden. Das erste und das zweite Schallereignis werden durch den Nutzer bewertet und mittels mindestens eines Umsetzungsfaktors wird das Hörsystem durch die Umsetzereinheit angepasst, sodass die Lautheit des ersten Schallereignisses vom Nutzer des Hörsystems gleich der Lautheit des zweiten Schallereignisses wahrgenommen wird. Erst durch die Verwendung benachbarter Frequenzen, beispielsweise im Abstand von 1 kHz (+/- 100 Hz), wird das Hörsystem des Nutzers verbessert konfiguriert.

Eine Vorrichtung zur Konfiguration eines nutzerspezifischen Hörsystems umfasst einen Schallgenerator zur Erzeugung von Schallereignissen (nachfolgend auch als Tonsignale bezeichnet) sowie eine Steuereinheit. Bevorzugt ist mindestens ein vom Nutzer des Hörsystems beabstandeter Lautsprecher zur Wiedergabe der Schallereignisse vorgesehen. Alternativ können die Schallereignisse auch durch das Hörsystem erzeugt und wiedergegeben werden. Die Steuereinheit veranlasst den Schallgenerator, der in bestimmten Anwendungen durch das Hörsystem gebildet sein kann, in einer vorbestimmten Abfolge mehrere Schallereignisse mit verschiedenen Frequenzen zu generieren. Dabei kann es sich um schmalbandige Sinussignale oder auch Tonsignale mit größerer Bandbreite handeln. Die generierten Schallereignisse werden gesteuert von der Steuereinheit vorbestimmt angepasst, insbesondere verstärkt, und zur Wiedergabe als Schallereignisse mit gleicher Lautheit an die Lautsprechereinheit gesendet bzw. direkt vom Hörsystem wiedergegeben. Die Steuereinheit empfängt über eine Bedienerschnittstelle Bewertungsdaten, die eine vom Nutzer wahrgenommene Lautheitsdifferenz zwischen im Frequenzbereich benachbarten widergegebenen Schallereignissen repräsentieren. Über die Bedienerschnittstelle wird der Steuereinheit somit signalisiert, ob der Nutzer die mit gleicher Lautheit widergegebenen Schallereignisse mit unterschiedlicher Frequenz gleich laut oder unterschiedlich laut wahrnimmt. Die Steuereinheit generiert daraufhin Korrekturwerte, die ausgegeben werden und am Hörsystem anwendbar sind, um dessen Umsetzereinheit so zu konfigurieren, dass die wahrgenommene Lautheitsdifferenz verringert oder vorzugsweise beseitigt wird.

Im Gegensatz zu einem System, wie es beispielsweise in der DE 10 2007 054 152 A1 beschrieben wurde, werden gemäß der vorliegenden Erfindung der oder die Lautsprecher in einer Entfernung von vorzugsweise 2 m zum Nutzer des Hörsystems aufgestellt. Ein Lautsprecher-Array ist für die Umsetzung der Erfindung nicht erforderlich, wenngleich möglich. Für die Ausführung der Erfindung ist es auch nicht erforderlich, ein Tonaudiogramm aufzunehmen. Während herkömmliche Systeme zumeist nur eine Feinanpassung der Hörsysteme zum Ziel haben, gestattet die Erfindung direkt die vollständige Konfiguration des Hörsystems. Im Gegensatz zum Stand der Technik ist ein vorherige Grundeinstellung des Hörsystems nicht erforderlich. Ebenso kann auf eine vorherige Messung der audiologischen Kenndaten verzichtet werden.

Bekannte Systeme, wie beispielsweise aus der DE 10 2011 104 536 A1 bekannt, nutzten Geräusche, die in der Nähe der Hörschwelle liegen. Bei einer Einstellung von Hörsystemen mit solchen schwellennahen Signalen besteht der Nachteil, dass viele Parameter im Hörsystem eine Verfälschung der Ergebnisse zur Folge haben können. Zudem ist ein Vergleich schwellennaher Töne untereinander für den Nutzer sehr schwierig. Stattdessen verwendet die vorliegende Erfindung deutlich überschwellige Tonsignale, die also für den normal hörenden Nutzer deutlich hörbar sind. Vorzugsweise werden Tonsignale mit einem Pegel von 45 - 80 dB genutzt.

Gemäß einer bevorzugten Ausführungsform werden vom Schallgenerator die Schallereignisse als Sinustöne, Terzen oder schmalbandiges Rauschen erzeugt.

Vorzugsweise umfasst die Bedienerschnittstelle eine Eingabeeinheit und eine Anzeigeeinheit, die beispielsweise in einem einzigen Gerät wie einem Tablet-PC zusammengefasst sein können.

Gemäß einer bevorzugten Ausführungsform umfasst die Vorrichtung eine temporäre Datenverbindung zwischen der Steuereinheit und dem Hörsystem, sodass die Korrekturwerte direkt auf das Hörsystem übertragen werden können. Die Datenverbindung kann kabelgebunden oder durch drahtlose Übertragungssysteme ausgebildet sein. Alternativ dazu können die Korrekturwerte natürlich auch über eine weitere Bedienerschnittstelle dem Hörsystem einprogrammiert werden.

Das erfindungsgemäße Verfahren zur Konfiguration eines nutzerspezifischen Hörsystems startet mit dem Schritt der Wiedergabe eines ersten Schallereignisses mit einer ersten Frequenz und einer vorbestimmten Lautheit an einem Lautsprecher, bevorzugt an einer mindestens zweikanaligen Lautsprechereinheit, die mindestens zwei voneinander sowie vom Nutzer des Hörsystems beabstandete Lautsprecher besitzt. Im nächsten Schritt erfolgt die Wiedergabe eines zweiten Schallereignisses mit einer zweiten, von der ersten Frequenz abweichenden, benachbarten Frequenz und derselben Lautheit wie beim ersten Schallereignis über die Lautsprechereinheit bzw. den einzelnen Lautsprecher. Unter Berücksichtigung einer vom Nutzer wahrgenommenen Lautheitsdifferenz zwischen den beiden Schallereignissen wird nachfolgend mindestens ein Umsetzungsfaktor der Umsetzereinheit des Hörsystems verändert, sodass die Lautheit des ersten Schallereignisses vom Nutzer des Hörsystems gleich der Lautheit des zweiten Schallereignisses wahrgenommen wird.

Das Ziel des Verfahrens ist erreicht, wenn alle vom Nutzer noch hörbaren Frequenzen an die Kurven gleicher Lautstärke angeglichen wurden.

Bevorzugt werden die zuvor genannten Schritte mehrfach wiederholt unter Anwendung abweichender, jeweils benachbarter Frequenzen der jeweils zu vergleichenden Schallereignisse, sodass das Hörsystem über einen vorgegebenen Frequenzbereich konfiguriert werden kann, sodass nachfolgend vom Nutzer sämtliche Schallereignisse mit derselben Lautheit wahrgenommen werden. Die jeweils miteinander verglichenen, benachbarten Schallereignisse sind voneinander jeweils um etwa 500 bis 1200 Hz beabstandet, d.h. sie unterscheiden sich vorzugsweise jeweils um etwa 1 kHz ± 200 Hz, besonders bevorzugt jeweils um 1 kHz ± 100 Hz, wobei selbstverständlich auch andere Differenzen in dem genannten Bereich möglich sind. Bevorzugt sind die Unterschiede entsprechend den Parameterbereichen des einzustellenden Hörsystems gewählt. Im tieferen Frequenzbereich, d.h. unterhalb von 1 kHz, sind die Abstände zwischen benachbarten Schallereignissen bevorzugt kleiner zu wählen, um auch in diesem Bereich noch mehrere Schallereignisse mit unterschiedlichen, benachbarten Frequenzen in die Konfigurationsschleife einbeziehen zu können. Im höheren Frequenzbereich, d.h. oberhalb von 6 kHz, sind die Abstände zwischen benachbarten Schallereignissen bevorzugt größer zu wählen, um in diesem Bereich mit einer überschaubaren Anzahl von Schallereignissen mit unterschiedlichen, benachbarten Frequenzen konfigurieren zu können.

Gemäß einer abgewandelten Ausführungsform erfolgt während der Konfiguration ein mehrfacher Wechsel zwischen den Frequenzen der wiedergegebenen Schallereignisse und/oder ein schrittweises Annähern der Lautheit benachbarter Frequenzen bis auf dasselbe Niveau.

In einer erweiterten Ausführungsform ist die Vorrichtung vollständig automatisiert. Dazu ist das Verfahren so angepasst, dass der Akustiker keine speziellen Eingaben am Hörsystem mehr machen muss. Stattdessen werden nur noch die Ergebnisse nach dem Lautheitsempfinden des Nutzers eingelesen und direkt im Hörsystem umgesetzt.

Um die Vorrichtung zu kalibrieren kann mit Hilfe eines Pegelmessers und eines Frequenzanalyzers der Raum, in welchem die Lautsprecher aufgestellt sind und die Schallereignisse wiedergegeben werden, mit einem weißen Rauschen eingemessen werden. Ziel ist dabei eine Angleichung der Lautsprecher an die Umgebung, sodass jeder Lautsprecher am Ende der Kalibrierung bis auf die üblichen Toleranzen genau die gleichen Pegel abgibt. Wird ein reduzierter Aufbau mit nur einem Lautsprecher verwendet, ist dieser genauso zu kalibrieren. Sind alle Lautsprecher dementsprechend eingepegelt, werden die einzelnen Ton-, bzw. Frequenzbänder mittels Pegelmesser vorzugsweise nach der Norm ISO 226:2003 eingepegelt. Ziel der Kalibrierung ist eine hohe Reproduzierbarkeit in allen Raumgrößen.

Gemäß einer nochmals abgewandelten Ausführungsform können die Schallereignisse auch durch das Hörsystem selbst erzeugt und wiedergegeben werden. Das dem Nutzer zur Verfügung gestellte Hörsystem kann somit selbst der Schallgenerator sein. In dieser Variante wird zwar auf das freie Schallfeld verzichtet, da die Wiedergabe der Schallereignisse über das Lautsprechersystem entfällt, jedoch führt dies zu einem besonders einfachen Aufbau der Vorrichtung.

Weitere Einzelheiten, Abwandlungen und Vorteile der vorliegenden Erfindung ergeben sich aus der nachfolgenden Beschreibung bevorzugter Ausführungsformen, unter Bezugnahme auf die Zeichnung. Es zeigen:
- Fig. 1:: eine Prinzipdarstellung einer ersten Ausführungsform einer Vorrichtung zur Konfiguration eines nutzerspezifischen Hörsystems;
- Fig. 2:: einen vereinfachten Ablaufplan einer ersten Ausführungsform eines erfindungsgemäßen Verfahrens zur Konfiguration eines nutzerspezifischen Hörsystems;
- Fig. 3a - 3c:: einen vereinfachten Ablaufplan einer zweiten Ausführungsform des erfindungsgemäßen Verfahrens;
- Fig. 4: eine Prinzipdarstellung einer zweiten Ausführungsform der Vorrichtung zur Konfiguration eines nutzerspezifischen Hörsystems.

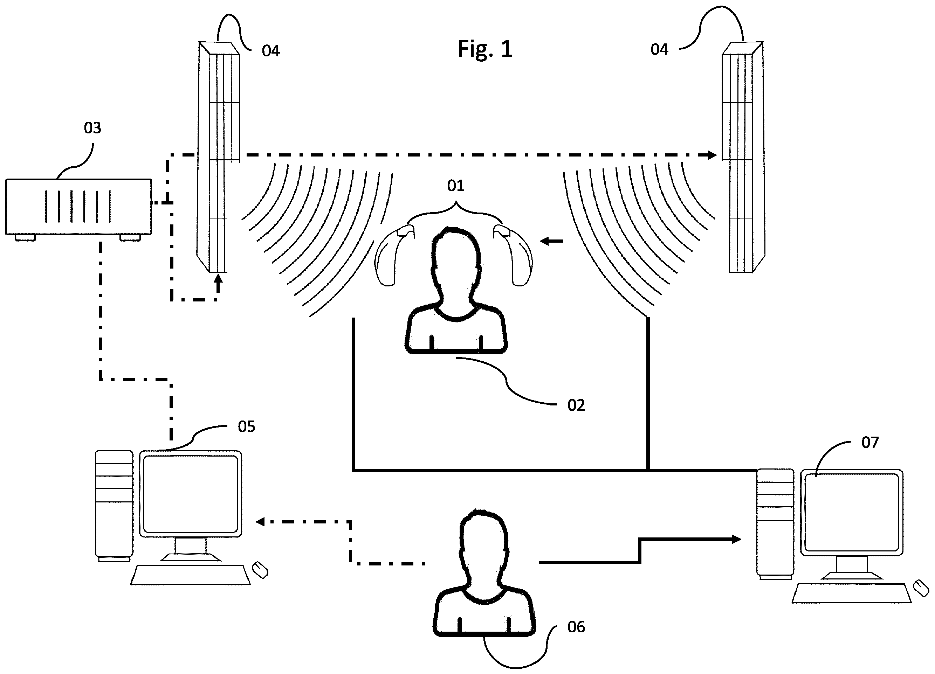

Die in Fig. 1 gezeigte Prinzipdarstellung veranschaulicht wesentliche Komponenten der Vorrichtung zur Konfiguration eines nutzerspezifischen Hörsystems sowie deren Zusammenwirken. Das nutzerspezifische Hörsystem 01, welches als solches nicht zur Vorrichtung gehört, umfasst ein oder zwei Hörgeräte als Teilsysteme, die jeweils eine Empfangseinheit zum Empfang von Schallereignissen, eine Umsetzereinheit zur frequenzabhängig variablen Anpassung der empfangenen Schallereignisse und eine Wiedergabeeinheit zur Wiedergabe der verstärkten Schallereignisse an das Hörorgan eines Nutzers 02 umfassen.

Beispielsweise handelt es sich bei dem Hörsystem 01 um sogenannte Deep-Fit-Otoplastiken oder Deep-Fit-CIC-Hörgeräte. Durch eine eingesetzte Otoplastik wird der Hörgang des Nutzers möglichst weitgehend verschlossen, so dass nur noch die vom Hörsystem 01 verstärkten Schallereignisse dem inneren Hörorgan des Nutzers 02 zugeführt werden. Die Umsetzereinheit ist bei solchen Systemen durch eine Verstärkungseinheit gebildet, welche die empfangenen Schallereignisse frequenzabhängig variabel verstärkt.

Es können aber auch abgewandelte Ausführungsformen von Hörsystemen konfiguriert werden. Die empfangenen Schallereignisse werden von solchen abgewandelten Hörsystemen nicht zwingend verstärkt sondern im weiteren Sinne verarbeitet/angepasst und beispielsweise als elektrische Signale an das Hörorgan geliefert.

Die Vorrichtung umfasst weiterhin einen Schallgenerator 03, welcher im dargestellten Ausführungsbeispiel aus einem Systemrechner sowie einem Verstärker besteht. Der Schallgenerator 03 erzeugt Schallereignisse (Tonsignale) und gibt diese an eine Lautsprechereinheit 04 weiter, durch welche Schallereignisse abgegeben werden. Die Lautsprechereinheit 04 ist in der dargestellten Ausführungsform zwei- oder mehrkanalig aufgebaut und besitzt hier mindestens zwei Lautsprecher, die voneinander beabstandet sowie beabstandet vom Nutzer 02 positioniert sind. Die Abstandsverhältnisse führen vorzugsweise zu einer für ein realistisches Stereo-Hörerlebnis geeigneten Position des Nutzers im freien Schallfeld. Der Nutzer 02 befindet sich somit im freien Schallfeld, so dass ihn die Schallereignisse wie in einer natürlichen Geräuschumgebung erreichen.

Bei abgewandelten, einfachen Ausführungsformen kann die Wiedergabe der Schallereignisse auch durch nur einen einzelnen Lautsprecher vorgenommen werden.

Schließlich umfasst die Vorrichtung eine Steuereinheit 05. Die Steuereinheit 05 veranlasst den Schallgenerator 03 in einer vorbestimmten Abfolge mehrere Tonsignale mit verschiedenen Frequenzen zu generieren. Die generierten Tonsignale werden vorbestimmt verstärkt und zur Wiedergabe als Schallereignisse mit gleicher Lautheit an die Lautsprechereinheit gesendet. Genormte Messverfahren zur Lautheitsmessung sind beispielsweise in DIN 45631 und ISO 532 B angegeben.

Im einfachsten Fall wird dem Nutzer somit ein erstes Schallereignis mit einer Frequenz von beispielsweise 2 kHz und einer Lautheit von 65 dB über die Lautsprechereinheit wiedergegeben und unmittelbar darauf ein zweites Schallereignis mit einer benachbarten Frequenz von 3 kHz und derselben Lautheit. Ein normal hörender Nutzer würde die beiden Schallereignisse als gleichlaut empfinden, während ein Nutzer mit einer Hörschädigung in dem betroffenen Frequenzbereich einen Unterschied in der Lautheit registriert. Die Schallereignisse werden gezielt oberhalb der Hörschwelle des Nutzers wiedergegeben, in einem Bereich normaler Umgebungslautstärke, beispielsweise mit 45 bis 80 dB. Eine vom Nutzer wahrgenommene Lautheitsdifferenz wird entweder vom Nutzer 02 oder von einem die Konfiguration durchführenden Hörgeräteakustiker 06 über eine Bedienerschnittstelle an der Steuereinheit 05 eingegeben. Die Steuereinheit 05 bestimmt aus den empfangenen Bewertungsdaten zur Lautheitsdifferenz Korrekturwerte und gibt diese aus, um die Umsetzereinheit des Hörsystems 01 so anzupassen, dass die Lautheitsdifferenz verringert oder vorzugsweise ganz beseitigt wird. Die Korrekturwerte können entweder unmittelbar in eine Programmiereinheit 07 eingespeist werden, welche die frequenzabhängige Verstärkung des Hörsystems 01 einstellt. Alternativ dazu können die Korrekturwerte dem Hörgeräteakustiker angezeigt werden, so dass dieser die entsprechenden Korrekturen über eine weitere Schnittstelle manuell in die Programmiereinheit 07 eingeben kann. Die Korrektur kann in mehreren Schritten sukzessive vorgenommen werden.

Eine nochmals abgewandelte Ausführungsform nutzt das Hörsystem selbst als Schallgenerator sowie für die Wiedergabe der Schallereignisse. Auf das Lautsprechersystem kann dabei verzichtet werden.

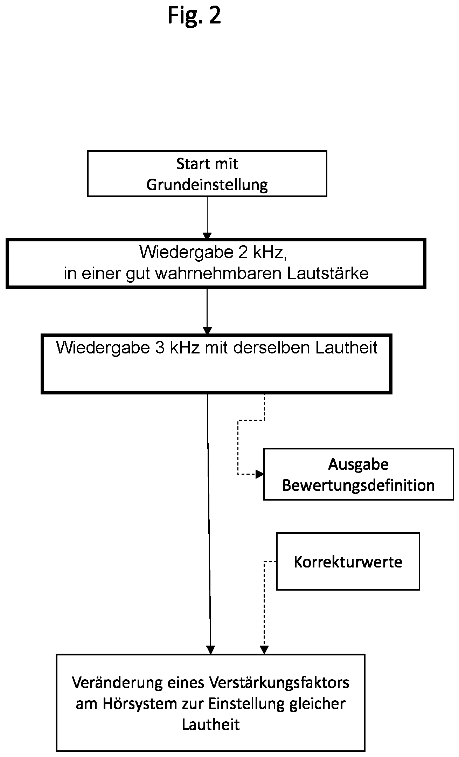

In Fig. 2 sind in Form eines Programmablaufplans die wesentlichen Schritte zur Ausführung eines erfindungsgemäßen Verfahrens zur Konfiguration eines nutzerspezifischen Hörsystems dargestellt. Vor dem Beginn der eigentlichen Konfiguration kann das Hörsystem mit der Grundeinstellung des Herstellers (First Fit) eingestellt werden, ohne zusätzliche Beaufschlagung mit frequenzabhängigen Verstärkungsparametern. Wenn der Nutzer an beiden Ohren mit Hörsystemen versorgt ist, werden diese verkoppelt und bei der zweikanaligen Lautsprechereinheit wird zunächst nur die Seite mit einem Schallereignis beaufschlagt, welche dem besser hörenden Ohr des Nutzers am nächsten liegt. Die eigentliche Konfiguration beginnt dann mit der Wiedergabe eines ersten Schallereignisses mit einer ersten Frequenz, beispielsweise 2 kHz, in einer gut wahrnehmbaren Lautstärke, beispielsweise mit einer Lautheit von 65 dB, zunächst nur auf einem Kanal der zweikanaligen Lautsprechereinheit, auf der Seite des besseren Hörvermögens des Nutzers. Unmittelbar darauf folgend wird ein zweites Schallereignis mit einer zweiten, benachbarten Frequenz, beispielsweise 3 kHz und derselben Lautheit wiedergegeben, ebenfalls auf der Seite des besseren Hörvermögens. Der Nutzer registriert entweder dieselbe Lautheit oder eine Lautheitsdifferenz trotz der objektiv bestehenden identischen Lautheit zwischen den beiden wiedergegebenen Schallereignissen. Über eine Bedienerschnittstelle kann eine wahrgenommene Lautheitsdifferenz in Form von Bewertungsdaten der Steuereinheit einer entsprechenden Vorrichtung eingegeben werden, im einfachsten Fall nur durch Auswahl des vom Nutzer als lauter empfundenen Schallereignisses.

Bei einer festgestellten Lautheitsdifferenz wird im nächsten Schritt ein Verstärkungsfaktor der Verstärkungseinheit (oder ein entsprechender Anpassungsparameter einer abgewandelten Umsetzereinheit) des Hörsystems verändert, so dass die Lautheit des ersten Schallereignisses vom Nutzer des Hörsystems gleich der Lautheit des zweiten Schallereignisses wahrgenommen wird. Das Hörsystem ist damit für den Frequenzbereich zwischen den Frequenzen der beiden wiedergegebenen, benachbarten Schallereignisse konfiguriert.

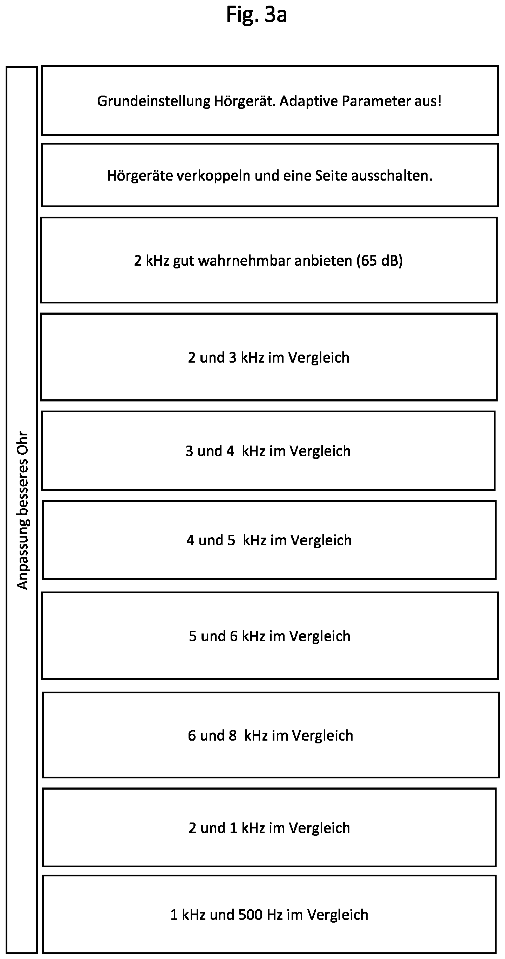

Vorzugsweise erfolgt die Konfiguration des Hörsystems nicht nur für zwei in ihren Frequenzen voneinander beabstandete, d.h. zueinander benachbarte Schallereignisse sondern für eine Mehrzahl von Schallereignissen, die jeweils um einen vorbestimmten Betrag in ihrer Frequenz voneinander abweichen, d.h. jeweils zueinander benachbart sind, z.B. mit einem Frequenzabstand jeweils im Bereich zwischen 500 und 1200 Hz. Fig. 3a - 3c zeigen eine entsprechend abgewandelte Ausführungsform des Verfahrens zur Konfiguration eines nutzerspezifischen Hörsystems, bei welchem die Konfiguration unter Verwendung von mehr als zwei Schallereignissen durchgeführt wird.

Der in Fig. 3a dargestellte Abschnitt "Anpassung besseres Ohr" entspricht den bereits erläuterten Schritten gemäß Fig. 2, wobei mehrere Schallereignisse mit unterschiedlichen Frequenzen genutzt werden. Die Hörgeräte des Nutzers werden zunächst in der Grundeinstellung betrieben, adaptive Parameter sind ausgeschaltet. Verwendet der Nutzer zwei Hörgeräte, werden diese verkoppelt und eine Seite (ein Teilsystem) wird ausgeschaltet. Im nächsten Schritt wird ein erstes Schallereignis mit beispielsweise 2 kHz, 65 dB wiedergegeben, sodass der Nutzer dieses erste Schallereignis mit einer deutlichen Lautheit wahrnimmt. Im nächsten Schritt wird dem Nutzer ein zweites Schallereignis mit einer benachbarten Frequenz von z.B. 3 kHz und derselben Lautheit wie das erste Schallereignis wiedergegeben. Der Nutzer kann die beiden Schallereignisse mit benachbarten Frequenzen und gleicher Lautheit direkt vergleichen und angeben, ob das zweite Schallereignis lauter oder leiser wahrgenommen wird. Im Falle einer Abweichung in der subjektiv wahrgenommenen Lautheit wird ein Korrekturwert / Verstärkungsfaktor angewandt, sodass der Nutzer dann etwa dieselbe Lautheit wahrnimmt. Die Auswahl des korrekten Verstärkungsfaktors kann ggf. sukzessive in mehreren Teilschritten erfolgen. Es folgen weiter Vergleich zwischen jeweils benachbarten Frequenzen, beispielsweise 3 und 4 kHz, 4 und 5 kHz, 5 und 6 kHz sowie 6 und 8 kHz, wobei jeweils Korrekturwerte für das Hörgerät ermittelt werden, die zur Wahrnehmung der jeweils zwei Schallereignisse benachbarter Frequenzen mit gleicher Lautheit führen. Weitere Schritte im tieferen Frequenzbereich durch Vergleich von Schallereignissen bei beispielsweise 2 und 1 kHz sowie 1 kHz und 500 Hz können folgen.

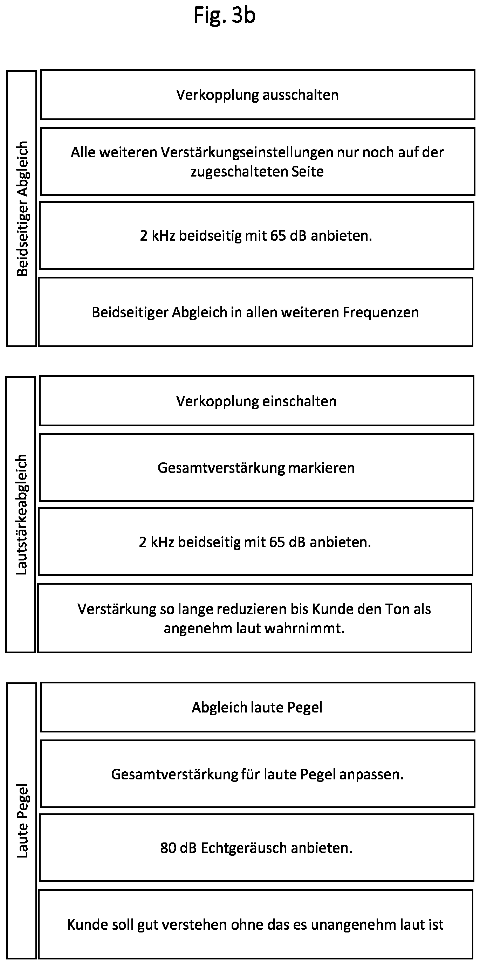

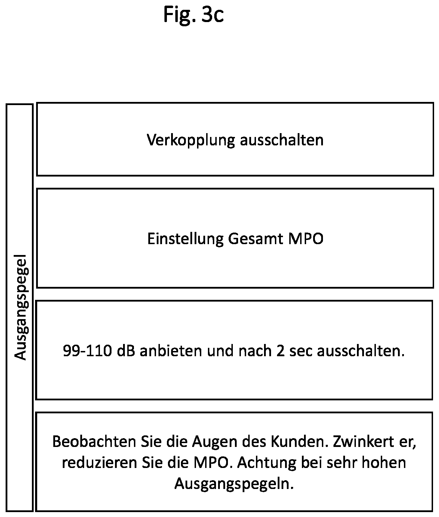

In den nachfolgenden Abschnitten des Ablaufplans nach Fig. 3b und 3c sind auch diejenigen Schritte dargestellt, die für die Konfiguration des zweiten Hörsystems (am zweiten Ohr) erforderlich sind (Abschnitt "Beidseitiger Abgleich") und die für eine abschließende Einstellung der Hörsysteme in Bezug auf das Einstellen der Wahrnehmungsschwelle und das Einstellen der maximalen Verstärkung der Hörsysteme zusätzlich ausgeführt werden können. Beide Hörsysteme des Nutzers werden vorzugsweise durch wechselseitiges Anbieten der Schallereignisse binaural aufeinander abgestimmt.

Wie aus Fig. 3b ersichtlich ist, wird im Abschnitt "Beidseitiger Abgleich" zunächst die Verkopplung der beiden Hörgeräte ausgeschaltet. Alle weiteren Verstärkungseinstellungen erfolgen in diesem Abschnitt nur noch auf der zugeschalteten Seite (Hörgerät). Dem hinzugeschalteten zweiten Hörgerät wird zuerst ein Schallereignis mit 2 kHz, 65 dB angeboten. Es folgt der Abgleich in allen weiteren Frequenzen, wie zuvor beschrieben wurde.

Es folgen abschließende Einstellungen im Abschnitt "Lautstärkeabgleich", im Abschnitt "Laute Pegel" und im Abschnitt "Ausgangspegel" (Fig. 3c).

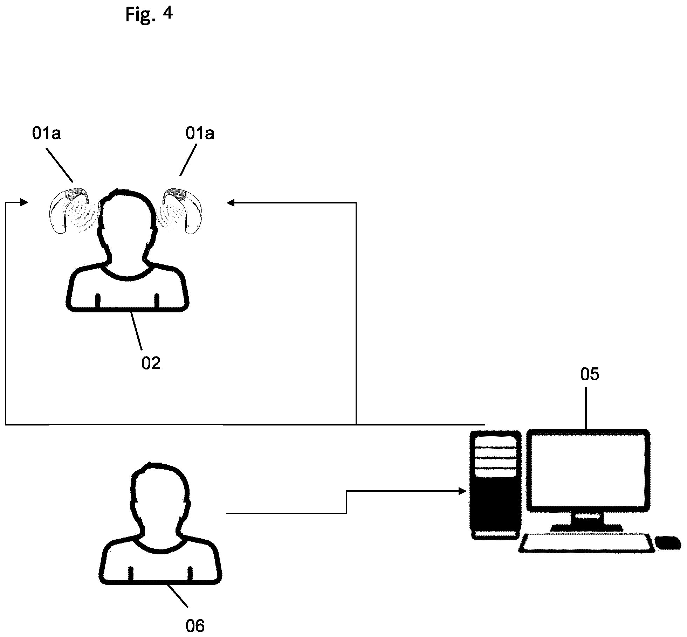

Fig. 4 zeigt eine abgewandelte Ausführungsform der Vorrichtung zur Konfiguration eines nutzerspezifischen Hörsystems 01a. Hierbei wird das Hörsystem selbst als Schallgenerator sowie für die Wiedergabe der Schallereignisse genutzt. Das angepasste nutzerspezifische Hörsystem 01a umfasst auch in diesem Fall ein oder zwei Hörgeräte als Teilsysteme, die jeweils eine Empfangseinheit zum Empfang von Schallereignissen, eine Umsetzereinheit zur frequenzabhängig variablen Anpassung der empfangenen Schallereignisse und eine Wiedergabeeinheit zur Wiedergabe der verstärkten Schallereignisse an das Hörorgan eines Nutzers 02 umfassen. Außerdem umfasst das Hörsystem 01a bzw. jedes Teilsystem eine integrierte Einheit, die als Schallgenerator arbeitet und von der Steuereinheit 05 aktiviert wird. Der integrierte Schallgenerator erzeugt Schallereignisse und gibt diese unmittelbar mittels der Wiedergabeeinheit des Hörsystems 01a wieder. Die Steuereinheit 05 veranlasst das Hörsystem in einer vorbestimmten Abfolge mehrere Tonsignale mit verschiedenen Frequenzen zu generieren und mit gleicher Lautheit wiederzugeben. Eine vom Nutzer 02 wahrgenommene Lautheitsdifferenz wird entweder vom Nutzer oder von einem die Konfiguration durchführenden Hörgeräteakustiker 06 über eine Bedienerschnittstelle an der Steuereinheit 05 eingegeben. Die Steuereinheit 05 bestimmt aus den empfangenen Bewertungsdaten zur Lautheitsdifferenz Korrekturwerte und gibt diese aus, um die Umsetzereinheit des Hörsystems 01 so anzupassen, dass die Lautheitsdifferenz verringert oder vorzugsweise ganz beseitigt wird. Die Korrekturwerte werden in diesem Fall direkt von der Steuereinheit 05, welche die Funktion der Programmiereinheit mit übernimmt, an das Hörsystem 01 geliefert.

### Bezugszeichenliste

- 01 -: Hörsystem
- 02 -: Nutzer
- 03 -: Schallgenerator
- 04 -: Lautsprechereinheit
- 05 -: Steuereinheit
- 06 -: Hörgeräteakustiker
- 07 -: Programmiereinheit

## Patentansprüche

1. Verfahren zur Konfiguration eines für einen Nutzer spezifischen Hörsystems (01), wobei das Hörsystem eine Empfangseinheit zum Empfang von Schallereignissen, eine Umsetzereinheit zur frequenzabhängig variablen Anpassung der empfangenen Schallereignisse und eine Wiedergabeeinheit zur Wiedergabe der angepassten Schallereignisse an das Hörorgan des Nutzers umfasst, wobei folgende Schritte ausgeführt werden:
a) Wiedergabe eines ersten Schallereignisses mit einer ersten Frequenz und einer vorbestimmten Lautheit über mindestens einen Schallerzeuger;
b) unmittelbar darauf Wiedergabe eines zweiten Schallereignisses mit einer zweiten Frequenz und einer für einen Normalhörenden selben Lautheit wie das erste Schallereignis über den Schallerzeuger, wobei die Frequenzen des ersten und des zweiten Schallereignisses zueinander benachbart sind;
c) im nächsten Schritt sukzessive Veränderung mindestens eines Anpassungsparameters der Umsetzereinheit des Hörsystems, wenn vom Nutzer eine Lautheitsdifferenz zwischen dem ersten und dem zweiten Schallereignis festgestellt wurde, sodass die Lautheit des ersten Schallereignisses vom Nutzer des Hörsystems gleich der Lautheit des zweiten Schallereignisses wahrgenommen wird.

2. Verfahren nach Anspruch 1, wobei die Schritte a) bis c) für weitere Schallereignisse mit anderen Frequenzen wiederholt werden, wobei die gewählten Frequenzen denjenigen Frequenzen entsprechen, für welche am Hörsystem frequenzspezifische Verstärkungsfaktoren einstellbar sind.

3. Verfahren nach Anspruch 1 oder 2, **dadurch gekennzeichnet, dass** die benachbarten Frequenzen des ersten und des zweiten Schallereignisses voneinander jeweils um etwa 500 bis 1200 Hz beabstandet sind, vorzugsweise um 1 kHz ± 200 Hz, besonders bevorzugt um 1 kHz ± 100 Hz beabstandet sind.

4. Verfahren nach einem der Ansprüche 1 bis 3, **dadurch gekennzeichnet, dass** die wiedergegebenen Schallereignisse Sinustöne, Terzen oder schmalbandiges Rauschen sind.

5. Verfahren nach einem der Ansprüche 1 bis 4, **dadurch gekennzeichnet, dass** als Schallerzeuger ein oder mehrere vom Nutzer des Hörsystems beabstandete Lautsprecher (04) oder das Hörsystem des Nutzers verwendet wird.

## Claims

1. A method for configuring a hearing system (01) specifically for a user, wherein the hearing system comprises a receiving unit for receiving sound events, a converter unit for the frequency-dependent variable adaption of the received sound events, and a reproduction unit for the reproduction of the adapted sound events to the hearing organ of the user, wherein the following steps are performed:
a) reproduction of a first sound event with a first frequency and a predetermined loudness via at least one sound generator;
b) immediately thereafter, reproduction of a second sound event with a second frequency and, for a normal hearing person, the same loudness as the first sound event via the sound generator, wherein the frequencies of the first and of the second sound event are adjacent to one another;
c) in the next step, successively changing at least one adaptation parameter of the converter unit of the hearing system if a loudness difference between the first and second sound event is detected by the user, so that the loudness of the first sound event is perceived by the user of the auditory system as equal to the loudness of the second sound event.

2. The method according to claim 1, wherein the steps a) to c) are repeated for other sound events with other frequencies, wherein the selected frequencies correspond to those frequencies for which frequency-specific amplification factors can be adjusted on the hearing system.

3. The method according to claim 1 or 2, wherein the adjacent frequencies of the first and of the second sound events are at a distance from one another of approximately 500 to 1200 Hz, preferably 1 kHz ± 200 Hz, especially preferably 1 kHz ± 100 Hz.

4. The method according to one of the claims 1 to 3, wherein the reproduced sound events are sinusoidal tones, thirds or narrow band noise.

5. The method according to one of the claims 1 to 4, wherein one or more loudspeakers (04) at a distance from the user of the hearing system or the hearing system of the user is used as sound generator.

## Revendications

1. Procédé de configuration d'un système auditif (01) spécifique à un utilisateur, sachant que le système auditif comprend une unité de réception pour recevoir des événements sonores, une unité de conversion pour l'adaptation variable en fonction de la fréquence des événements sonores reçus et une unité de restitution pour restituer à l'organe auditif de l'utilisateur les événements sonores adaptés, sachant que les étapes suivantes sont exécutées :
a) restitution d'un premier événement sonore avec une première fréquence et d'une intensité sonore prédéterminée par le biais d'au moins un générateur de son,
b) directement ensuite, restitution d'un deuxième événement sonore avec une deuxième fréquence et d'une même intensité pour un entendant normal comme le premier événement sonore par le biais du générateur de son, sachant que les fréquences du premier et du deuxième événement sonore sont voisines l'une de l'autre,
c) dans l'étape suivante, modification successive d'au moins un paramètre d'adaptation de l'unité de conversion du système auditif, lorsqu'une différence d'intensité sonore a été constatée par l'utilisateur entre le premier et le deuxième événement sonore de telle manière que l'intensité sonore du premier événement sonore est perçue par l'utilisateur du système auditif de façon égale à l'intensité sonore du deuxième événement sonore.

2. Procédé selon la revendication 1, sachant que les étapes a) à c) sont répétées pour d'autres événements sonores avec d'autres fréquences, sachant que les fréquences choisies correspondent aux fréquences pour lesquelles des facteurs d'amplification spécifiques à la fréquence peuvent être réglés sur le système auditif.

3. Procédé selon la revendication 1 ou 2, **caractérisé en ce que** les fréquences voisines du premier et du deuxième événement sonore sont respectivement espacées l'une de l'autre d'environ 500 à 1200 Hz, de préférence de 1 kHz ± 200 Hz, en particulier espacées de préférence de 1 kHz ± 100 Hz.

4. Procédé selon l'une quelconque des revendications 1 à 3, **caractérisé en ce que** les événements sonores restitués sont des tons sinusoïdaux, des tierces ou des bruits à bande étroite.

5. Procédé selon l'une quelconque des revendications 1 à 4, **caractérisé en ce qu'**un ou plusieurs haut-parleurs (04) à distance de l'utilisateur du système auditif ou le système auditif de l'utilisateur sont utilisés en tant que générateurs de son.
